# EUROPEAN PATENT SPECIFICATION

(11) **EP 0 418 142 B1**
(45) Date of publication and mention of the grant of the patent: **23.03.1994**
(21) Application number: 90402495.7
(22) Date of filing: 11.09.1990
(51) Int. Cl.: C07C 11/02, C07C 1/20

(54) **Process for synthesis of lower olefins from methanol**
Verfahren zur Synthese von niedrigen Olefinen aus Methanol
Procédé pour la synthèse d'oléfines inférieures à partir de méthanol

(30) Priority: 14.09.1989 JP 237082/89
(43) Date of publication of application: 20.03.1991
(73) Proprietor: Showa Shell Sekiyu Kabushiki Kaisha, Tokyo (JP); Inui, Tomoyuki, Uji-shi Kyoto 611 (JP)
(72) Inventor: Inui, Tomoyuki, Uji-shi, Kyoto 611 (JP)
(74) Representative: Bourgognon, Jean-Marie

(56) References cited:
- EP-A- 0 249 915
- WO-A-86/04577

## Description

The present invention relates to a process for producing lower olefins mainly composed of ethylene from methanol. Especially, it relates to a process in which conversion reaction of methanol to lower olefins is carried out with a high selectivity for ethylene of about 90%.

Selective synthesis of lower olefins from methanol have been studied as a main task of C₁ chemistry.

EP-A-249915 mentions synthesis of lower olefins from methanol in the presence of silica-alumino phosphat catalyst containing Co or Fe.

In an attempt to synthesize olefins rich in ethylene at high selectivity, the inventor also has made development of various shape selective catalysts which make it possible to synthesize the olefins at temperatures from moderate temperature (about 400°C) to low temperature (about 300°C).

Catalysts prepared by ion exchanging chabazite which are used for synthesis of lower olefins from methanol have the defect of short life because of their too strong acidity and coking thereof. [For example, B.P.Pat.2061999A, R.G.Anthong.B.B. Singh. Hydrocarbon Processing, March 85 (1981)]. When ZSM-34 catalyst is used, ethylene content in the resulting lower olefins is at most 50% and besides, the catalyst deteriorates rapidly. [Mobil. Japanese Patent Kokai No.53-58499 (1978)]. In the case of ZKU-4 catalyst, life of the catalyst can be prolonged. [T.Inui. Y.Takegami. Hydrocarbon Processing, Nov. 117 (1982), Japanese Patent Kokai No.57-63135 (1982)].

On the other hand, the inventors have succeeded in producing lower olefins containing ethylene and propylene at a ratio of about 50% using metallo-silicate catalyst (Fe-Si) developed by them. [T.Inui. et al. J. Catl. 98, 491 (1986)].

As a result of the inventor's research on catalyst of chabazite structure, it has been found that Ni-containing silica-alumino phosphate catalyst (hereinafter referred to as "Ni-SAPO catalyst") (atomic ratio Si/Ni: 20-100) increases selectivity for ethylene in conversion reaction of methanol to lower olefins and besides, has a long life. Thus, the present invention has been accomplished.

That is, the present invention relates to a process for synthesis of lower olefins mainly composed of ethylene from methanol which comprises carrying out conversion reaction of methanol-containing gas as a starting material at a reaction temperature of 250-600°C and a GHSV of 1000-8000 h⁻¹ in the presence of an Ni-SAPO catalyst (the atomic ratio Si/Ni: 20-100).

Fig. 1 is a graph which shows relation between reaction temperature and reaction products.

Fig. 2 is a graph which shows change of reaction products with time when reaction temperature was 450°C.

Fig. 3 is a graph which shows change of reaction products with time when reaction temperature was 600°C.

The range of 20-100 of Si/Ni atomic ratio of Ni-SAPO catalyst is the range determined considering acidity of the catalyst. That is, in the course of production of lower olefins, especially, ethylene from methanol, various reactions such as production of dimethyl ether by dehydration and dimerization of methanol, production of ethylene by dehydration of dimethyl ether, production of olefin by polymerization of ethylene, production of aromatic hydrocarbons and saturated heydrocarbons by decomposition of olefin, cyclization and isomerization of olefin, and coking of the aromatic hydrocarbon take place as successive reactions and simultaneous reactions.

Acidity of the catalyst is adjusted by employing the Si/Ni atomic ratio of the above range and as a result, the reaction of production of ethylene selectively takes place as a main reaction among the above various reactions and besides, coking of aromatic hydrocarbon is controled resulting in prolongation of catalyst life.

Reaction conditions are reaction temperature: 250-600°C and GHSV (gas hourly space velocity): 1000-8000 h⁻¹. GHSV is selected so that optimum conversion can be obtained at the reaction temperature. At a reaction temperature of 250-600°C, the optimum GHSV in conversion reaction of from methanol to ethylene is 1000-8000 h⁻¹.

Analysis of gas produced is carried out by gas chromatography. Analysis of C₁-C₄ hydrocarbons is carried out by column VZ-10, 3 mm⌀ x 3 m at 90°C and analysis of gasoline fraction is carried out by column OV-101, 0.25 mm⌀ x 30 m at 150°C using hydrogen flame ion detector (FID).

Furthermore, analysis of methanol and dimethyl ether is carried out by Porapack-T column, 2 mm⌀ x 3 m at 120°C and analysis of CO and CO₂ is carried out by active carbon column, 3 mm x 3 m at 100°C using a thermal conductivity detector (TCD).

As carrier gas, He gas is used at flow rate of 30 cc/min for FID and Ar gas is used at a flow rate of 30 cc/min for TCD.

The present invention will be explained by the following examples.

### Catalyst:

Synthesis of Ni-SAPO catalyst used in the present invention was carried out by rapid crystallization method. [T.Inui et al. Pros. 8th Inten. Congr. Catal. Berlin III-569 (1984)].

That is, silica (CATHALOID 30), aluminum isopropoxide, phosphoric acid and nickel sulfate were used as starting materials for Ni, Si, Al and P, respectively at an atomic ratio Ni:Si:Al:P of 7.5 x 10⁻²:0.3:1.0:1.0. The catalyst was synthesized by rapid crystallization method using tetraethylammonium hydroxide as a crystallizing agent at 200°C for 4 hours. This catalyst was calcined at 600°C in air stream and the resulting crystal powder was tabletd and ground to 7-15 meshes and used for reaction.

Surface area of the catalyst was 403 m²/g according to BET method.

### Reaction:

0.214 g of Ni-SAPO catalyst (Si/Ni atomic ratio: 40) obtained above was packed in a quartz tube of 6 mm in inner diameter. Volume of the catalyst which occupied the reaction tube was 1.1 ml. The catalyst was standardized by passing nitrogen gas therethrough for 30 minutes at 450°C.

Reaction was carried out using a mixed gas of MeOH 20%-N₂ 80% (vol%) as starting material gas at a passing rate of GHSV 2000 h⁻¹ and at a reaction temperature in the range of 250-600°C with changing the temperature every 50°C.

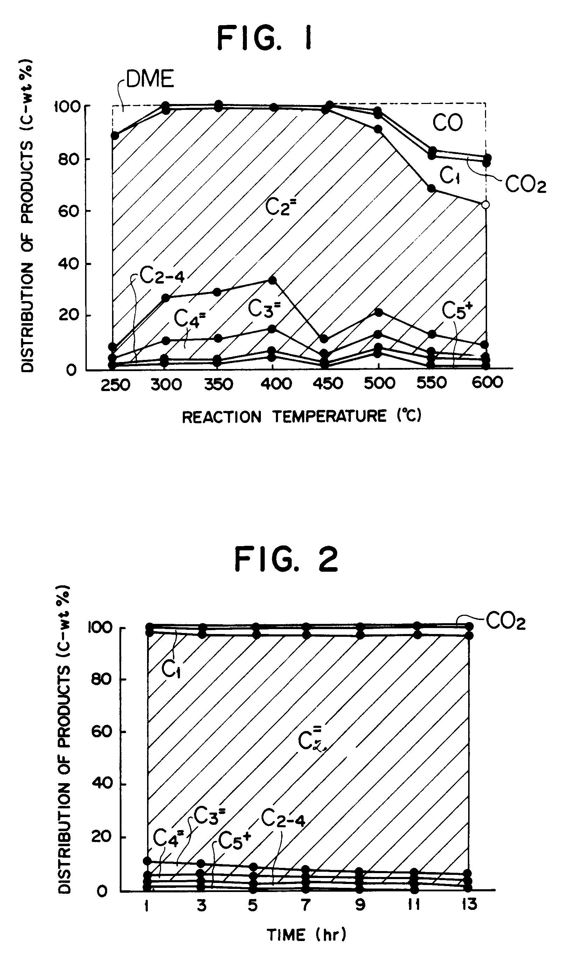

After lapse of 1 hour of passing the reaction gas, the products were analyzed and the results are shown in Fig. 1.

It can be seen from Fig. 1 that conversion rate of methanol was 100% for all temperatures used and methanol was wholly converted to hydrocarbons at temperatures up to 350-450°C and most of the hydrocarbons were C₂-C₄ olefins and especially, selectivity for ethylene was high. At 450°C, selectivity for ethylene was 88,0%, that for propylene was 5.3% and that for butene was 2.5% and thus, total selectivity for olefin reached 95.8%.

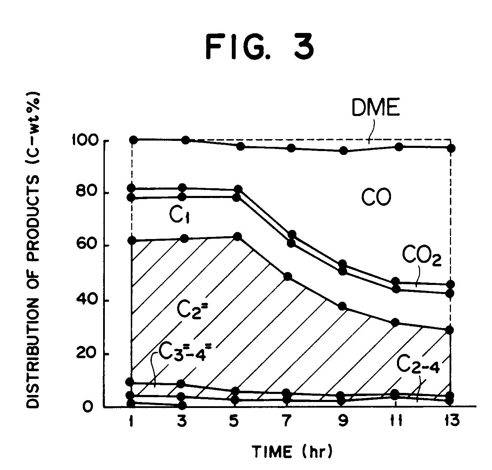

Further, change of conversion rate with time was examined over 13 hours at reaction temperatures of 450°C and 600°C. The results are shown in Fig. 2 (reaction temperature 450°C) and in Fig. 3 (reaction temperature 600°C). Conversion rate of methanol was 100% in both cases. It can be seen from Fig. 2 that at the reaction temperature of 450°C, methanol was converted to ethylene very stably and in nearly quantitatively and production of C₃-C₄ olefins decreased with increase in passing time and after lapse of 13 hours, selectivity for ethylene reached 91.0%.

It can be seen from Fig. 3 that at the reaction temperature of 600 °C, catalyst activity decreased after lapse of 5 hours.

In synthesis of ethylene from methanol, Ni-SAPO catalyst with chabacite structure showed markedly high ethylene selectivity which has not been able to be attained by chabacite type zeolite catalysts.

As explained above, ethylene selectivity of about 90% can be attained in synthesis of lower olefins from methanol using Ni-SAPO catalyst.

## Claims

1. A process for synthesis of lower olefins mainly composed of ethylene from methanol or methanol containing gas which comprises carrying out the conversion reaction of methanol or methanol-containing gas in the presence of Ni-containing silica-alumino phosphate catalyst having a chabazite structure and a Si/Ni atomic ratio of 20-100 at a reaction temperature of 250-600°C and at a GHSV of 1000-8000 h⁻¹.

## Patentansprüche

1. Verfahren zur Herstellung von hauptsächlich Ethylen enthaltenden niederen Olefinen aus Methanol oder Methanol-enthaltenden Gasen, dadurch gekennzeichnet, daß man die Umsetzung des Methanols oder der Methanolenthaltenden Gase in Gegenwart eines Nickel-enthaltenden Kieselsäure-Aluminophosphat-Katalysators mit einer Chabazit-Struktur und einem Si:Ni-Atomverhältnis von 20 bis 100 zu 1 bei einer Reaktionstemperatur von 250 bis 600°C und einer GHSV von 1000 bis 8000 h⁻¹ durchführt.

## Revendications

1. Procédé de synthèse d'oléfines inférieures composées principalement d'éthylène à partir du méthanol ou de gaz en contenant, qui consiste à effectuer la réaction de conversion du méthanol ou de gaz en contenant en la présence de catalyseurs de silice-aluminophosphate contenant du Ni ayant une structure de chabazite et un rapport atomique Si/Ni de 20 à 100 à une température réactionnelle de 250 à 600°C et à une VSHG de 1000 à 8000 h⁻¹.
